(19) Europäisches Patentamt
European Patent Office
Office européen des brevets

(11) Veröffentlichungsnummer: **0 129 719**
**B1**

(12) # EUROPÄISCHE PATENTSCHRIFT

(45) Veröffentlichungstag der Patentschrift: **17.11.88**

(51) Int. Cl.⁴: **C 08 F 220/54**, C 12 N 11/08

(21) Anmeldenummer: **84105909.0**

(22) Anmeldetag: **24.05.84**

(54) **Makroporöse Perlpolymerisate, Verfahren zu ihrer Herstellung und ihre Anwendung.**

(30) Priorität: **28.05.83 DE 3319506**
**06.02.84 DE 3404021**

(43) Veröffentlichungstag der Anmeldung: **02.01.85 Patentblatt 85/1**

(45) Bekanntmachung des Hinweises auf die Patenterteilung: **17.11.88 Patentblatt 88/46**

(84) Benannte Vertragsstaaten: **AT BE CH DE FR GB IT LI LU NL SE**

(56) Entgegenhaltungen:
**DE - A - 1 795 235**
**DE - A - 2 722 751**

**DECHEMA MONOGRAPHAS,no. 1724-1731, vol. 84, Characterization of Immobilized Biocatalysts (file pages 49/50)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Noetzel, Siegfried, Dr., An den Römergärten 3, D-6233 Kelkheim (Taunus) (DE)**
Erfinder: **Mauz, Otto, Dr., Heidestrasse 21, D-6237 Liederbach (DE)**
Erfinder: **Sauber, Klaus, Dr., Falkenstrasse 35, D-6232 Bad Soden am Taunus (DE)**

**EP 0 129 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, biologisch aktive Substanzen, wie Enzyme, Antikörper, Antigene, Hormone udgl. unter Erhalt ihrer Aktivität an polymere Trägermaterialien über kovalente Bindungen zu fixieren, um auf diesem Weg beispielsweise Enzyme zu stabilisieren, zu reinigen oder wasserunlöslich zu machen. Solchermassen immobilisierte biologisch aktive Substanzen bieten erhebliche Vorteile gegenüber der löslichen Form: Zum einen ist die Abtrennbarkeit durch Sedimentation nach Beendigung einer Reaktion vereinfacht, zum anderen ist die Stabilität und Wiederverwendbarkeit der Präparate um ein Vielfaches erhöht.

In der DE-B-2 237 316 werden als Trägersubstanzen quellbare, vernetzte Perlpolymerisate beschrieben, die durch Copolymerisation von reaktiven Gruppen enthaltenden Monomeren, vernetzenden Monomeren und hydrophilen Monomeren erhalten werden. Als reaktive Gruppe werden dabei die Halogenalkyl-, die Epoxid-, die Carbonsäurechlorid-, Carbonsäureanhydrid-, Carbonsäureazid-, Carbonsäurephenylester- und Hydroxamsäure-Gruppe offenbart. Diese Trägermaterialien haben jedoch eine Reihe von Nachteilen; so ist die Fixierung der biologisch aktiven Substanzen bei einigen von ihnen ziemlich langwierig; ihre Aktivität ist teilweise unbefriedigend und bei Verwendung der Anhydridvarianten kommt es ausserdem zur Einführung von Ladungen.

Aus der DE-A-2 102 514 ist es bekannt, in ein hydrophiles Polymeres Oxirangruppen einzuführen, die dann zur Bindung eines biologisch wirksamen Stoffes verwendet werden können. Unter den hydrophilen Polymeren sind auch solche genannt, die Acrylamidgruppen enthalten. Diesen Trägern fehlt jedoch die perlförmige Morphologie und die poröse Struktur. Dadurch sind sie beispielsweise für die Anwendung in einem Säulenverfahren nicht geeignet.

Die Aufgabe vorliegender Erfindung bestand darin, ein polymeres Material, insbesondere als Trägermaterial für biologisch aktive Substanzen auf Basis von (Meth)Acrylamid-Derivaten bereitzustellen, das die Nachteile des Standes der Technik nicht besitzt und insbesondere Perlform und eine ausreichende Porosität aufweist. Eine weitere Aufgabe bestand darin, ein hierfür geeignetes Verfahren zu entwickeln.

Zur Lösung dieser Aufgabe schlägt die Erfindung ein Polymerisat vor, bestehend im wesentlichen aus

a) Einheiten, die sich von mindestens einem Monomeren der Formel

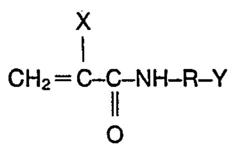 (I),

ableiten, in der X Wasserstoff oder Methyl ist, R einen aliphatischen Kohlenwasserstoffrest mit 1 bis 12 Kohlenstoffatomen bedeutet und Y für OH oder $NH_2$ steht, und aus Einheiten, die sich von weiteren Monomeren ableiten, die mit Monomeren der Formel (I) copolymerisierbar sind, wobei die mittlere Teilchengrösse der Polymerisat-Teilchen im Bereich von 20 bis 800 µm liegt, dadurch gekennzeichnet, dass als Einheiten von weiteren Monomeren solche vorhanden sind, die sich

b) von mindestens einem Monomeren mit hydrophilen Gruppen und

c) von mindestens einem Monomeren mit vernetzenden Gruppen ableiten und dass die Polymerisat-Teilchen im wesentlichen kugelförmige Gestalt und einen mittleren Porendurchmesser von 5 bis 2000 nm aufweisen.

Weiterhin betrifft die Erfindung ein Verfahren zur Herstellung derartiger Polymerisate durch Polymerisieren von a) Verbindungen der Formel

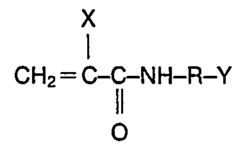 (I),

in der X, R und Y die obige Bedeutung haben, mit mindestens einem damit copolymerisierbaren weiteren Monomeren, wobei die Polymerisation in einem flüssigen Dispersionsmittel, das unter den Polymerisationsbedingungen die Monomeren und das Polymerisat nicht löst, in Gegenwart eines radikalisch wirksamen Initiators und eines Dispersionsstabilisators durchgeführt wird, dadurch gekennzeichnet, dass als weitere Monomere b) mindestens ein Monomer mit hydrophilen Gruppen und c) mindestens ein Monomer mit vernetzenden Gruppen eingesetzt werden und dass als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen α-Olefin mit 8 bis 30 C-Atomen verwendet wird.

Die Erfindung hat weiterhin die Verwendung der so erhaltenen Polymere, vorzugsweise nach Umsatz mit Spacern, als Trägermaterialien zur Herstellung von trägergebundenen biologisch aktiven Substanzen zum Gegenstand.

Das erfindungsgemässe Polymerisat enthält die sich von den Monomeren gemäss Formel (I) ableitenden Einheiten, zweckmässigerweise in Mengen von 5 bis 90 Mol-%, vorzugsweise 10 bis 80 Mol-% und insbesondere 15 bis 60 Mol-%, bezogen auf das Gesamtpolymere. Grössere und kleinere Mengen als die vorstehend angegebenen sind grundsätzlich möglich, jedoch ist dies im Regelfall mit Nachteilen verbunden. Die optimale Menge innerhalb der vorstehend angegebenen Bereiche hängt u.a. ab von der angestrebten Besetzungsdichte, von dem Molekulargewicht der biologisch aktiven Substanz.

Der Rest R in der obigen Formel (I) hat, wie bereits erwähnt, die Bedeutung eines aliphatischen Kohlenwasserstoffrestes mit 1 bis 12 Kohlenstoffatomen, der linear, verzweigt oder cyclisch (cycloaliphatisch) sein kann. Bevorzugt ist

der Rest R ein Alkylenrest mit 1 bis 6 Kohlenstoffatomen. Beispielhaft seien hierfür genannt: Methylen, Äthylen, Propylen, Isopropylen, n-Butylen, i-Butylen, Pentylen, Hexylen, 2-Äthylhexylen, Cyclopentylen, Cyclohexylen, Methylencyclohexyl und dergleichen. Der Rest Y steht in obiger Formel (I) bevorzugt für die OH-Gruppe, wobei diese wiederum bevorzugt primär ist. Entsprechende Monomere gemäss Formel (I) sind demnach N-Methylol(meth)acrylamid, N-(2-Hydroxyäthyl)-(meth)acrylamid, N-(3-Hydroxypropyl)(meth)-acrylamid, N-(2-Hydroxypropyl)(meth)acrylamid, N-(2-Aminoäthyl)(meth)acrylamid, N-(3-Amino-propyl)(meth)acrylamid und N-(6-Aminohexyl)-(meth)-acrylamid). Es können auch Gemische derartiger Monomerer eingesetzt werden.

Zumindest ein Teil, vorzugsweise zumindest 0,5 Mol-% und insbesondere 1 bis 40 Mol-% des Restes Y ist in dem erfindungsgemässen Polymerisat mit sogenannten Spacern umgesetzt. Im allgemeinen wird die Menge an Spacer etwa 0,1 Mol-% bis 20 Mol-%, vorzugsweise 1 bis 10 Mol.-%, bezogen auf die Gesamtmonomer-Einheiten, im erfindungsgemässen Perlpolymeren nicht übersteigen.

Unter Spacern sind Verbindungen zu verstehen, die sowohl mit dem Rest Y der (–CONH–R–Y)-Seitenkette des erfindungsgemässen Perlpolymerisates reagieren als auch eine reaktive Gruppe, vorzugsweise eine Epoxygruppe, einführen, die dann mit der biologisch aktiven Substanz reagieren kann. Zumindest ein Teil des Restes Y ist also nach dem Umsatz mit dem Spacer durch die Gruppierung

$$Y'–B–Z \qquad (II)$$

ersetzt, worin Y′ gleich O oder NH ist, B für einen organischen Rest (den Spacer im engeren Sinne), insbesondere für einen Kohlenwasserstoffrest mit 1 bis 12 C-Atomen steht, der gegebenenfalls durch Heteroatome wie O, NH und S unterbrochen sein kann, und Z eine funktionelle Gruppe darstellt, die mit der biologisch aktiven Verbindung eine kovalente Bindung eingehen kann. Hierzu gehören beispielsweise die Gruppen

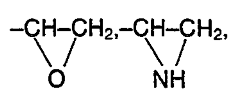

–COX (X = H, Halogen, $-N_3$, –OR; R = Alkylrest mit 1 bis 6 C-Atomen), $-CH(OR)_2$ (R wie vorstehend),

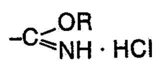

(R wie vorstehend), $-N_2]^+$ oder –NCO.

Vorzugsweise bedeutet Y′ gleich Sauerstoff, B einen aliphatischen insbesondere unverzweigten Kohlenwasserstoffrest mit 1 bis 6 C-Atomen, einen Arylrest oder einen Alkylarylrest und Z gleich

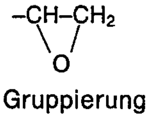

Beispiele für die Gruppierung Y′–B–Z sind:

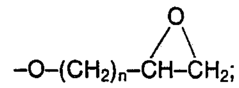 n = 1–8

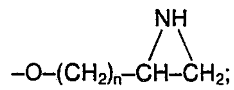 n = 1–8

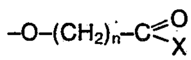 n = 1–8
X = H, OH, Halogen, $N_3$, OR

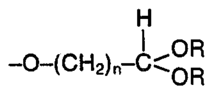 n = 1–6
R = Alkylrest mit 1–6 C-Atomen

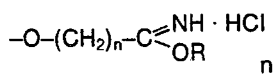
n = 1–6
R = Alkylrest mit 1–6 C-Atomen

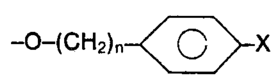
X = $NH_2$, $N_2]^+$, NCO

Das Perlpolymerisat enthält erfindungsgemäss noch mindestens eine weitere Monomereinheit, die sich von einem mit dem Monomeren gemäss der Formel (I) copolymerisierbaren Monomeren ableitet. Vorzugsweise handelt es sich hierbei um zwei weitere voneinander verschiedene Monomereinheiten.

Eine Monomereinheit davon leitet sich von einem Monomeren mit hydrophilen Gruppen ab, das dem als Träger eingesetzten Polymeren ausreichende Hydrophilie und ausreichende Quellbarkeit in Wasser verleiht. Dies ist insofern von Bedeutung, als die Verankerungsreaktion mit der biologisch aktiven Substanz zumeist im wässrigen System durchgeführt wird und die hydrophile, biologisch aktive Substanz an das Trägermaterial herandiffundieren können muss. Zu diesem Monomeren gehören beispielsweise die in der DE-A-2 237 316 als Komponente c) aufgeführten Monomeren. Im Rahmen der vorliegenden Erfindung werden dabei bevorzugt: N-Vinylpyrrolidon, (Meth)-Acrylamid, (Meth)Acrylsäurealkylester mit jeweils 2 bis 6 C-Atomen in der Alkylgruppe, Hydroxyalkylester der (Meth)Acrylsäure mit 2 bis 6 C-Atomen in der Alkylgruppe, N-Vinyl-N-alkyl-acetamid ($C_1$–$C_4$-Alkyl), Vinylacetat oder Vinylencarbonat. Letztere beiden Monomeren verleihen allerdings erst nach entsprechender Verseifung dem Polymerisat hydrophile Eigenschaften. Gegebenenfalls können auch mehrere dieser hydrophilen Monomer-Einheiten vorhanden sein.

Das Verhältnis von Monomer gemäss Formel (I) zu hydrophiler Komponente ist dabei auch abhängig von der Art des zu bindenden Enzyms. Bei sehr hohen Molekulargewichten des Enzyms bzw. des Substrates, das mit dem Enzym reagieren

soll, ist es zweckmässig, das Molverhältnis zu Gunsten der hydrophilen Komponente zu erhöhen, da aus sterischen Gründen benachbarte durch Derivatisierung der Hydroxylgruppen erhaltene bindungsfähige Spacergruppen nicht zur Reaktion gelangen und evtl. sogar störend wirken können. Weiterhin richtet sich die Menge an hydrophiler Komponente auch nach der Menge der erfindungsgemäss bevorzugt vorhandenen vernetzenden Komponente. Je grösser deren Menge ist, desto grösser wird in der Regel auch die erforderliche Menge an hydrophiler Komponente sein, um dem Trägerpolymeren ausreichende Hydrophilie und Quellbarkeit zu vermitteln. Im allgemeinen beträgt erfindungsgemäss die Menge an hydrophiler(n) Monomer-Einheit(en) im Polymeren 5 bis 70 Mol.-%, vorzugsweise 20 bis 50 Mol.-% und insbesondere 30 bis 50 Mol-%, bezogen auf das Polymere. Eine ausreichende Hydrophilie des Polymerisates ist auf jeden Fall dann gegeben, wenn dieses Polymerisat – ohne Mitverwendung eines Vernetzers (siehe nachfolgend) – wasserlöslich wäre. Eine ausreichende Quellbarkeit in Wasser liegt im Regelfall dann vor, wenn das Polymerisat – nach Vernetzung – bis auf das 30-fache, vorzugsweise auf das 3- bis 10-fache seines ursprünglichen Schütt-Volumens anquillt.

Als weitere mit dem Monomer gemäss Formel (I) copolymerisierbare Verbindung enthält das erfindungsgemässe Polymere vernetzende Monomer-Einheiten, wie sie aus dem Stand der Technik bekannt sind. Als typische Vertreter seien hier genannt: Divinyläther von Glykolen wie Äthylenglykoldivinyläther, Butandioldivinyläther, Hexandioldivinyläther, N,N'-Alkylenbis(meth)acrylamide mit bis zu 12 C-Atomen, vorzugsweise bis zu 6 C-Atome enthaltenden geradkettigen oder verzweigten Alkylenresten wie N,N'-Methylenbisacrylamid, N,N'-Äthylenbisacrylamid, N,N'-Hexamethylenbisacrylamid, N,N'-Methylenbismethacrylamid, N,N'-Äthylenbismethacrylamid, N,N'-Hexamethylenbismethacrylamid, N,N'-Äthylidenbisacrylamid, Glyoxalbisacrylamid, (1,2-Bisacrylamido-1,2-dihydroxyäthan), Bis-acrylamidoessigsäure, Äthylenglykol-bis-methacrylsäureester, Butandiolbismethacrylsäureester, Triallylcyanurat, Trisacryloylperhydrotriazin, Divinylbenzol, Adipinsäuredivinylester, N,N'-Divinylethylenharnstoff, N,N'-Divinylpropylenharnstoff, Ethyliden-bis-3-(N-vinylpyrrolidon), N,N'-Divinyldiimidazolyl(2,2') und 1,1'-Bis(3,3'-vinylbenzimidazolin-2-on)-1,4-butan, Vinylacrylat, Allylmethacrylat u.a. Es können auch mehrere verschiedene vernetzende Monomereinheiten vorhanden sein. Einzelne dieser Vernetzer, z.B. der N,N'-Divinylethylenharnstoff, N,N'-Divinylpropylenharnstoff, Ethyliden-bis-3-(N-vinylpyrrolidon), N,N'-Divinyldiimidazolyl(2,2') und 1,1'-Bis(3,3'-vinylbenzimidazolin-2-on)-1,4-butan, oder das N,N'-Methylenbisacrylamid können auch zur Hydrophilie des Polymeren beitragen.

Die Menge an vernetzender Monomer-Einheit und damit die Vernetzungsdichte hängt dabei von der Anwendung ab. Bei Enzymreaktionen im Rührkessel oder für Diagnostika kann eine geringe Vernetzungsdichte vorteilhaft sein; wird dagegen eine Säulenfüllung vorgenommen, ist eine hohe Formstabilität der Perlpolymerisate und damit eine hohe Vernetzungsdichte Voraussetzung. Je nach Anwendungsart kann daher die Menge an vernetzender Monomer-Einheit bis zu 60 Mol-%, bezogen auf das Polymere, betragen. Vorzugsweise liegt sie zwischen 1 und 50 Mol.-% und insbesondere zwischen 5 und 40 Mol-%. Wie hierzu bereits weiter oben ausgeführt, steht die Menge an vernetzender Komponente mit der Menge der hydrophilen Komponente in gewisser Relation. Zumeist wird man die Menge an vernetzendem Monomeren so wählen, dass das Perlpolymerisat in Tetrahydrofuran bis auf das 14-fache, vorzugsweise das 0- bis 8-fache seines ursprünglichen Schüttvolumens aufquillt.

Wasserlösliche Träger sind dann von Interesse, wenn die Umsetung des Trägers mit der biologisch aktiven Substanz in einer wässrigen Lösung des Trägers vorgenommen werden soll.

Gegebenenfalls kann ein unvernetztes Trägerpolymeres in bekannter Weise auch durch nachträgliche chemische Umsetzung vernetzt werden, z.B. durch Diisocyanate. Das Polymere sollte in diesem Fall einen etwas höheren Anteil an sich vom Monomeren gemäss Formel I ableitenden Einheiten enthalten.

Als gegebenenfalls weitere nicht hydrophile und nichtvernetzende Monomer-Einheiten können beispielsweise solche vorhanden sein, die sich ableiten von: Acryl- und Methacrylsäureester mit 5–12 C-Atomen im Alkylrest, (Meth)Acrylnitril, Vinylester mit 4–18 C-Atomen in dem Carbonsäurerest, wie Vinylbutyrat, Vinylstearat, und Vinylester verzweigter Carbonsäuren mit 10 bis 12 C-Atomen; weiterhin Vinylaromaten, wie Styrol oder α-Methylstyrol. Diese Monomer-Einheiten können im Polymeren in Mengen von 4 bis 40 Mol-%, vorzugsweise 8 bis 20 Mol-%, bezogen auf das Polymere, vorhanden sein.

Das erfindungsgemässe Perlpolymere besteht aus überwiegend kugelförmigen Teilchen, deren mittlere Teilchengrösse im trockenen, ungequollenen Zustand 20 bis 800 μm, vorzugsweise 50 bis 300 μm beträgt und die vorzugsweise eine enge Teilchengrössenverteilung aufweisen. Das jeweilige Optimum der Teilchengrösse hängt dabei vor allem von dem speziellen Einsatzgebiet ab. Bei einem ohne Druck durchgeführten Säulenverfahren wird man beispielsweise die Teilchengrösse innerhalb der vorstehend genannten Grenzen entsprechend grösser wählen können als bei einem Druckverfahren. Die Perlen des erfindungsgemässen Perlpolymerisates sind überwiegend als makroporöse Perlen ausgebildet. Dies drückt sich in dem sich daraus ergebenden erfindungsgemässen mittleren Porendurchmesser im Bereich von 5 bis 2000 nm, vorzugsweise 10 bis 1000 nm aus.

Die Bestimmung des Porendurchmessers (Porenvolumens) erfolgt in der Weise, dass zunächst das Porenvolumen gemäss der Kapillardruckmethode (Quecksilberporosimetrie) bestimmt wird (vgl. hierzu «Ullmanns Encyclopädie der technischen Chemie», Bd. 5 (1980), S. 751–752). Daraus

ergibt sich dann der mittlere Porendurchmesser durch Berechnung nach der auf Seite 752, linke Spalte oben dieser Literaturstelle angegebenen Gleichung. Daneben ist eine Porengrössenbestimmung auch durch Rasterelektronenmikroskopie möglich.

Die erfindungsgemässen Perlpolymerisate eignen sich insbesondere als Träger für biologisch aktive Substanzen. Sie können aber auch für andere Zwecke eingesetzt werden, beispielsweise als Ionenaustauscher und Adsorptionsmittel für chromatographische Verfahren.

Das erfindungsgemässe Verfahren zur Herstellung dieser Perlpolymeren wird unter den üblichen bekannten Bedingungen der Perlpolymerisaten durchgeführt, wie sie beispielsweise in der DE-A-2 237 316 oder der DE-OS 2 556 759 beschrieben sind, jedoch mit der Neuerung, dass spezielle Dispersionsstabilisatoren zum Einsatz kommen.

Bei diesen handelt es sich um vorzugsweise alternierende Copolymere aus Maleinsäureanhydrid und einem Vinylalkyläther, vorzugsweise einem Vinyl-n-alkyläther mit 6 bis 30 C-Atomen, vorzugsweise 10 bis 20 C-Atomen in der Alkylgruppe, oder einem Vinylester mit 6 bis 30 C-Atomen, vorzugsweise 10 bis 20 C-Atomen in der Carbonsäuregruppe oder einem längerkettigen α-Olefin mit 8 bis 30 C-Atomen, vorzugsweise 10 bis 20 C-Atomen. Als Beispiele für derartige Vinylalkyläther, Vinylester bzw. längerkettigen α-Olefinen seien hier genannt: Vinyloctyläther, Vinyldecyläther, Vinyldodecyläther, Vinylstearyläther, Vinylmyricyläther, Ethylhexansäure-Vinylester, Isononansäure-Vinylester, Versaticsäure-Vinylester, Laurinsäure-Vinylester, Stearinsäure-Vinylester und Vinylester verzweigter Carbonsäuren mit 10 is 12 C-Atomen; Octen-(1), Decen-(1), Dodecen-(1), Tetradecen-(1), Octadecen-(1), Tricosen-(1).

Diese Dispersionsstabilisatoren sind bereits in Mengen von 0,001 Gew.-%, bezogen auf die Gesamtmenge an Monomeren, wirksam. Zumeist werden Mengen von 0,005 bis 10 Gew.-%, vorzugsweise 0,01–5 Gew.-% (bezogen auf Gesamtmenge an Monomeren) verwendet.

Die reduzierte spezifische Viskosität (RSV) dieser als Dispersionsstabilisatoren eingesetzten Copolymeren liegt in der Regel zwischen 0,01 und 1,0 dl/g, (bestimmt in 0,6%iger Lösung in Toluol bei 25 °C). Der entsprechende Vorzugsbereich beträgt bei den Copolymeren aus Maleinsäureanhydrid und Vinylalkyläther bzw. Vinylester 0,05 bis 1,0 dl/g und bei den Copolymeren aus Maleinsäureanhydrid und längerkettigem α-Olefin 0,01 bis 0,1 dl/g. Das Molverhältnis zwischen Maleinsäureanhydrid und dem Vinylalkyläther bzw. Vinylester oder dem längerkettigen α-Olefin liegt im allgemeinen zwischen 1:4 bis 1:1, vorzugsweise zwischen 1:2 bis 1:1 und insbesondere bei 1:1.

Als radikalisch wirksame Initiatoren kommen erfindungsgemäss solche in Betracht, die in der Monomerphase gut und in dem flüssigen Dispersionsmittel möglichst wenig löslich sind. Beispiele hierfür sind organische Peroxide, wie Di-tert.-butylperoxid, Dibenzoylperoxid, Bis(o-Methylbenzoyl)peroxid, tert.-Butylhydroperoxyd, Cumolhydroperoxid, Di-isopropylperoxidicarbonat, Cyclohexanonperoxid oder aliphatische Azoverbindungen, wie α,α'-Azodiisobuttersäurenitril, Azobis-cyanvaleriansäure, 1,1'-Azo-cyclo-hexan-1,1'-dicarbonsäurenitril und Azodicarbonamid. Bei Verwendung von Wasser oder Gemischen von Wasser mit wasserlöslichen Stoffen als Inertmittel werden wasserlösliche Initiatoren, wie Ammonium-, Natrium- und Kaliumperoxydisulfat, Cyclohexylcarbonatokaliumsulfatoperoxyd, Bernsteinsäureperoxyd und tert.-Butylpermaleinat eingesetzt. Gegebenenfalls können auch entsprechende Redoxysysteme Verwendung finden. Die Menge an Initiator beträgt zumeist 0,01 bis 5, vorzugsweise 0,1 bis 2 Gew.-% (bezogen auf die Gesamtmenge der Monomeren).

Als flüssige Dispersionsmittel zur Durchführung der erfindungsgemässen Perlpolymerisation dienen vor allem solche organischen Verbindungen, die unter Normalbedingungen flüssig sind, einen Siedepunkt von oberhalb 60 °C, vorzugsweise im Bereich von 85–300 °C, aufweisen und welche die Monomeren, das Polymere und vorzugsweise auch den Initiator unter den Polymerisationsbedingungen nicht oder jedenfalls nur spurenweise lösen, um die unerwünschte Emulsionspolymerisation zu unterbinden. Gut geeignet sind beispielsweise Kohlenwasserstoffe mit 6 bis 20, vorzugsweise 12 bis 16 Kohlenstoffatomen, insbesondere Paraffine. Auch ein Gemisch von verschiedenen Verbindungen kann als Dispersionsmittel eingesetzt werden. Geeignete Kohlenwasserstoffe oder Kohlenwasserstoffgemische sind z. B. n-Hexan, n-Heptan, n-Octan, Cyclohexan, iso-Oktan, Benzinfraktionen mit Siedebereichen zwischen 90 und 170 °C und Paraffinöl dünnflüssig (Deutsches Arzneibuch, 7. Ausgabe, DAB 7). Das Verhältnis der Monomerphase zur Dispersionsmittelphase kann in weiten Grenzen variieren, beispielsweise zwischen 1:1 bis 1:50, vorzugsweise 0,5:1 bis 1:15 (Gewichtsverhältnis).

Um möglichst hohe Porosität der Perlpolymerisate zu erreichen, werden dem Polymerisationssystem oder vorzugsweise den Monomeren vorzugsweise bestimmte inerte, flüssige Komponenten (Inertmittel) zugesetzt. Hierunter sollen solche Stoffe verstanden sein, in denen sich die Monomeren lösen oder mit ihnen mischbar, andererseits aber im Dispergiermittel unlöslich und damit mit diesem nicht mischbar sind. Nach ihrem Verhalten zu den entsprechenden Copolymeren kann man die Inertmittel in Quellungs- und/oder Fällungsmittel einteilen. Bei einer hydrophilen Matrix werden polare Inertmittel in der Regel eine Quellung begünstigen, wie z. B. Dimethylformamid, Dimethylsulfoxid, Dioxan, Wasser etc., während sich unpolare Substanzen, wie Glycerintriacetat etc., als Fällungsmittel für das Copolymerisat erweisen. Das optimale Inertmittel bzw. Inertmittelgemisch lässt sich durch einige einfache Routineversuche leicht ermitteln. Insbesondere, wenn Perlpolymere mit relativ geringem Vernetzungsgrad angestrebt werden, dürfte es sich empfehlen, eine Mischung aus polarem und unpolarem Inertmittel

einzusetzen. Die Inertmittel nehmen an der Polymerisation nicht teil, werden jedoch vom Polymerisat umhüllt und bei der Aufarbeitung wieder herausgelöst. Dadurch entstehen permanente Poren. Die Porengrösse ist durch Art und Menge des Inertmittels beeinflussbar, hängt aber auch von der Menge an vernetzender Komponente ab.

Die Inertmittel können allein oder in Mischung eingesetzt werden. Als Beispiele seien genannt: Methanol und seine höheren Homologen, Äthylenglykol, Methylglykol, Propylglykol, Diäthylenglykol, Triäthylenglykol, Butandiol(1,4), Glycerin, Polyäthylenglykole, Polypyrrolidone, Diäthylenglykol-dimethylether, Glycerin-triacetat, Ethylencarbonat, Formamid, Dimethylformamid, Dimethylsulfoxid, Dioxan und Wasser.

Die Menge an zugesetztem Inertmittel ist weitgehend variabel. Sie hängt u.a. von der Monomerzusammensetzung des Trägers, insbesondere seines Gehaltes an Vernetzer, der erwünschten Porosität (Porengrösse) sowie vom genauen Verwendungszweck des Trägerpolymeren ab. So wird sich bei einem hohen Vernetzungsgrad eine entsprechend grosse Menge an Inertmittel empfehlen, um eine bestimmte Porosität (Porengrösse) zu erreichen. Bei ein und demselben Vernetzungsgrad wird die Porosität (Porengrösse) gleichfalls umso grösser sein, je mehr an Inertmittel eingesetzt wird. Naturgemäss lässt sich dies nur innerhalb bestimmter Grenzen steigern, da ansonsten die Wandstärke der makroporösen Perlen und damit deren mechanische Festigkeit zu gering wird. In den meisten Fällen wird eine Menge an Inertmittel, die dem 0,02- bis 5-fachen, vorzugsweise dem 0,04–3-fachen der Menge an eingesetzten Monomeren entspricht, zufriedenstellende Ergebnisse liefern.

Das Monomere gemäss Formel (I) sowie das weitere (bzw. die weiteren) Comonomere(n) werden in solchen Mengen eingesetzt, dass ein Polymeres mit den weiter oben angegebenen Mengen an Monomer-Einheiten resultiert. Hierzu wird das Monomere gemäss Formel (I) in der Regel in Mengen von 5 bis 90 Mol-%, vorzugsweise 10 bis 80 Mol-%, bezogen auf das Gesamtmonomerengemisch, verwendet. Die Menge an hydrophilen Monomeren beträgt demgegenüber zumeist 5 bis 70 Mol-%, vorzugsweise 20 bis 50 Mol-%, bezogen auf das Gesamtmonomerengemisch, und die Menge an vernetzendem Monomeren – falls eingesetzt – bis zu 60 Mol-%, vorzugsweise 1 bis 50 Mol-%, bezogen auf die Gesamtmonomerenmenge.

Die Herstellung der Monomeren gemäss der Formel (I) erfolgt in bekannter Weise, zum Beispiel durch Umsatz von (Meth)acrylsäurechlorid mit Verbindungen der Formel $H_2N–R–Y$ in Gegenwart geeigneter Säureakzeptoren.

Das erfindungsgemässe Verfahren wird zweckmässigerweise in einem mit einer Rührvorrichtung versehenen Reaktionsgefäss bei Temperaturen von zumeist 20 bis 150 °C, vorzugsweise 65 bis 125 °C durchgeführt. Die Teilchengrösse des Perlpolymerisates wird in bekannter Weise durch die Rührgeschwindigkeit und das Phasenverhältnis eingestellt. Besonders vorteilhaft ist die Verwendung eines senkrecht stehenden, zylindrischen Gefässes mit flaschem Boden, das mit einem koaxial angebrachten Rührer versehen ist, dessen Welle bis fast auf den Gefässboden reicht. Das Reaktionsgefäss ist vorzugsweise vakuumfest und kann mit Rückflusskühler, Zulauftrichter, Gaseinleitungsrohr und Temperaturmessgerät versehen werden. Die Beheizung und Kühlung des Gefässes erfolgt im allgemeinen durch ein Flüssigkeitsbad, z.B. ein Ölbad oder Wasserbad.

Es ist vorteilhaft, das erfindungsgemässe Verfahren unter Ausschluss von Luftsauerstoff durchzuführen. Das Reaktionsgefäss wird daher vor Beginn mit einem inerten Gas, vorzugsweise Stickstoff, gespült.

Nach Beendigung der Polymerisationsreaktion werden die nicht umgesetzten Monomeren aus dem Reaktionsgefäss entfernt z.B. durch Verdampfen bei vermindertem Druck, vorzugsweise einem Druck von 13.3322 bis 1999.83 Pa (0,1 bis 15 Torr). Nach der Entfernung der Restmonomeren wird das Dispersionsmittel vom festen Polymeren abgetrennt, z.B. durch Dekantieren, Filtrieren oder Absaugen des Überstandes. Anschliessend wird das Polymerisat, falls erforderlich, mit leichtsiedenden organischen Lösungsmitteln, z.B. einem Kohlenwasserstoff, einem niederen Alkohol oder Aceton gewaschen und schliesslich getrocknet. Die Trocknung des Polymerisates erfolgt bei einer Temperatur von zumeist 20 bis 100 °C, vorzugsweise von 40 bis 80 °C; eine Trocknung unter vermindertem Druck ist dabei empfehlenswert.

Bei Einsatz des erfindungsgemässen Perlpolymerisates als Trägermaterial für biologisch aktive Substanzen, wird dieses vorzugsweise zunächst – wie bereits erwähnt – mit Spacern umgesetzt. Als Spacer kommen erfindungsgemäss die hierfür bekannten homo- und hetero-bifunktionellen Verbindungen in Frage, deren zweite funktionelle Gruppe die Kopplung mit der zu fixierenden biologisch aktiven Substanz übernimmt (vgl. die DE-C-2 421 789 und 2 552 510, sowie Ullmanns Encyclopädie der technischen Chemie, 4. Auflage, Bd. 10, S. 540 und «Characterization of Immobilized Biocatalysts», Verlag Chemie, Weinheim, 1979, S. 53). Vorzugsweise werden erfindungsgemäss als Spacer Verbindungen eingesetzt, die Epoxidgruppen einführen, wie Epichlorhydrin oder dessen Homologe (α-β′-Epoxy-ω-halogenalkane) sowie Diepoxyde wie Äthylenglykol-1,2-diglycidyläther und Butandiol-1,4-diglycidyläther.

Die Reaktion beispielsweise mit den epoxydgruppenhaltigen Spacern wird in an sich bekannter Weise unter Anwendung überschüssiger Mengen an Epoxydverbindungen im allgemeinen während zwei bis sechs Stunden bei Temperaturen von 50 bis 200 °C, vorzugsweise in Gegenwart von basischen Katalysatoren wie tertiären Aminen, Alkalien, Dimethylformamid usw. und gegebenenfalls unter Verwendung von inerten Verdünnungsmitteln wie Dioxan usw. durchgeführt. Die Isolierung der epoxydierten Polymerteilchen erfolgt in gleichfalls bekannter Weise durch Absaugen und Auswaschen mit niedrig siedenden, die Polymerteilchen nicht angreifenden organischen Lösemitteln, wie Aceton oder Diäthyläther. Die inerten organischen Waschmittel werden dann bei 40 bis 60 °C und unter vermindertem Druck (26.66 KPa ≙ 200 mm Hg) unter Überleiten von Stickstoff im Vakuumtrockenschrank entfernt.

Überraschenderweise zeigt es sich, dass die so erhaltenen epoxydgruppenhaltigen Polymeren eine beträchtliche höhere Aktivität aufweisen als die entsprechend der DE-AS 2 237 316 durch direkte Polymerisation von epoxydgruppenhaltigen Monomeren erhaltenen Produkte.

Unter dem Begriff «biologisch aktive Substanzen» seien die bekannten in vivo oder in vitro wirksamen natürlichen oder künstlich hergestellten Stoffe verstanden, wie Enzyme, Aktivatoren, Inhibitoren, Antigene, Antikörper, Vitamine, Hormone, Effektoren, Antibiotika und Proteine. Der letztere Begriff umfasst dabei auch Proteine mit bestimmten Nicht-Proteinsubstituenten wie Metallionen, Polysacchariden, Porphyringruppen, Adenindinucleotid, Ribonucleinsäure und Phospholipide. Auch Polypeptidfragmente, z.B. die aktiven Teile von Enzymmolekülen, fallen unter den Begriff biologisch aktive Substanzen.

Von den vorstehend genannten biologisch aktiven Substanzen sind erfindungsgemäss die Enzyme bevorzugt. Beispiele für Enzyme sind Adenyldesaminase, Alkohol-Dehydrogenase, Asparaginase, Carboxypeptidase, Chymotrypsin, Diphosphoesterase, α-Glucosidase, Glucose-Isomerase, Glucose-Oxidase, Glucose-6-phosphat-Dehydrogenase, Hexokinase, Invertase, β-Lactamase, Lactase, Lactat-Dehydrogenase, versch. Lectine, NAD-Kinase, Neuraminidase, Papain, Peroxidase, Phosphatasen (alkalisch und sauer), 5′-Phosphodiesterase, Pyruvant Kinase, Ribonuclease, Trypsin.

Beispiele für andere biologisch aktive Substanzen sind Hormone, wie Insulin und die verschiedensten Hypophysen-Hormone, Proteine der gamma-Globulinfraktion, z.B. Antikörper der Klasse G, M, A, D und E, andere Blutfaktoren, z.B. Antihämophiliefaktor, die Blutgerinnungsfaktoren, spezielle Antikörper, z.B. Hepatitis-, Poliomyelitis-, Finnen-, Mumps-, Influenza- oder Kaninchenantikörper, Antigene, wie Hepatitis-, Polyomyelitis-, Finnen-, Mumps-, Influenca- oder Kaninchenantigene zur Reinigung oder Stimulierung geeigneter Antikörperreaktionen, wobei das Antigen (nach dem Unlöslichmachen) in der unlöslichen Form verbleibt und folglich nicht in den Körper eindringen und diesen schädigen kann, so wie allgemeine Körperproteine, wie Hämoglobin oder Albumin.

Die Verankerungsreaktion zwischen der biologisch aktiven Substanz wird in bekannter Weise durchgeführt, wie etwa in der DE-A-2 407 340 oder in den DE-C-2 215 687, 2 421 789 und 2 552 510 beschrieben. Zumeist wird die Umsetzung bei Raumtemperatur oder darunter liegenden Temperaturen durchgeführt. Letzteres insbesondere dann, wenn die zu verankernde biologisch aktive Substanz von Hause aus instabil ist; in diesem Fall liegen dann die Temperaturen unterhalb von +10 °C, vorzugsweise bei 0 bis +5 °C.

Die Verankerungsreaktion erfolgt vorzugsweise in der Umgebung eines neutralen pH-Wertes, beispielsweise bei pH-Werten von 5 bis 9, da hier die meisten biologisch aktiven Substanzen am stabilsten sind. In der Regel ist es auch nicht erforderlich, stärker saure oder alkalische Bedingungen einzuhalten, da die erfindungsgemässen makroporösen Perlpolymerisate auch bereits im Neutralbereich mit den meisten der in Frage kommenden Substanzen schnell reagieren. Die dabei entstehende Bindung bietet genügend Stabilität für lange Lagerungen und hohe Operationsstabilität.

Pro 1 Gewichtsteil der zu verankernden biologisch aktiven Substanz werden vorzugsweise etwa 10 bis 80 Gewichtsteile des Trägerpolymeren, insbesondere eines solchen, in welchem mindestens 50 Mol-% Einheiten gemäss Monomeren der Formel (I) enthalten sind, verwendet. Grössere Mengen des Trägerpolymeren können zweckmässigerweise in den Fällen eingesetzt werden, in denen die Einheiten gemäss Monomeren der Formel (I) weniger als 50 Mol-% des Polymeren ausmachen.

Die Erfindung wird nachfolgend anhand von Beispielen näher erläutert.

Herstellung eines Dispersionsstabilisators (Copolym. aus Maleinsäureanhydrid und Vinylstearyläther)

In einem Rührkolben wurden 98 g Maleinsäureanhydrid (1 Mol) und 296 g Vinylstearyläther (1 Mol) in 250 ml Aceton vorgelegt, 5 ml Diisopropylperkarbonat (40%ige Lösung in Phthalat) zugesetzt und das Gemisch unter Rühren und Stickstoff 5 Stunden bei 60 °C polymerisiert.

Nach Abkühlen wurde das ausgefallene Produkt abgesaugt und mehrmals mit Aceton gewaschen.

Das Molverhältnis der beiden Monomeren im Copolymeren betrug 1:1; der RSV-Wert lag bei 0,224 dl/g (gemessen in 0,6%iger Lösung in Toluol bei 25 °C).

I. Herstellung der erfindungsgemässen Polymerträger

Beispiel 1

Copolymerisat aus N-Methylolacrylamid, N-Vinylpyrrolidon und N,N′-Methylenbisacrylamid)

In einem Rundkolben mit Rührer, Thermometer, Stickstoffeinleitrohr und Rückflusskühler wurden 800 ml dünnflüssiges Paraffinöl DAB 7 (Dispergiermittel), 2,0 g eines Copolymerisates aus Maleinsäureanhydrid und Octadecen-(1), (Molverhältnis 1:1; RSV-Wert 0,064 dl/g, gemessen in 0,6%iger Lösung in Toluol bei 25 °C), 33,3 g N-Methylolacrylamid, 36,7 g N-Vinylpyrrolidon, 30 g N,N′-Methylenbisacrylamid, 2 g Azo-diisobuttersäurenitril, 212 ml Dimethylformamid und 90 ml Polyäthylenglykol (Molekulargewicht etwa 400) vorgelegt.

Dieses Gemisch wurde dann unter Rühren langsam hochgeheizt. Bei ca. 65 °C begann die exotherme Polymerisationsreaktion, wobei die Temperatur auf ca. 80 °C anstieg. Diese Temperatur wurde mittels eines thermostatisierten Ölbades 1 Stunde gehalten, dann wurde zur Auspolymerisation die Badtemperatur vier Stunden auf 90 °C erhöht. Anschliessend wurde das Heizbad entfernt und der Ansatz unter Rühren auf 40 °C abkühlen gelassen. Danach wurde die Rührung abgestellt, worauf sich nach einiger Zeit das perlförmige Polymerisat absetzte. Die Hauptmenge des Paraffinöls wurde dann abgehebert und anschliessend der Rest über eine Nutsche abgesaugt. Das erhaltene Polymerisat wurde anschliessend unter Rühren mit Petrolether behandelt, um das anhaftende Paraffinöl zu entfernen. Anschliessend wurde mit Methanol, dann mit Aceton ausgerührt und schliesslich mit Aceton bei Siedetemperatur extrahiert, um die nicht umgesetzten Monomeren sowie den Dispergator herauszulösen. Schliesslich wurde das Polymerisat im Vakuumschrank über Nacht bei 50 °C getrocknet und gesiebt.

Die Ausbeute betrug 95 g (= 95% der Theorie) vernetztes Copolymerisat.

Im wesentlichen das gleiche Produkt wird erhalten, wenn ein entsprechendes Dispersionsmittel aus Maleinsäureanhydrid und Dodecen eingesetzt wird.

Es wurden 15 g der Siebfraktion 50–100 µm 16 Stunden in 100 ml Epichlorhydrin gequollen, dann vier Stunden auf 115 °C erhitzt und nach dem Abkühlen auf 25 °C abgesaugt. Dann wurde zweimal mit jeweils 200 ml Aceton während 30 Minuten verrührt, abgesaugt und über Nacht im Vakuumtrockenschrank bei 40 °C unter Stickstoffüberlagerung aufbewahrt.

Die Auswaage ergab 14,6 g perlförmiges Produkt mit einem Schüttgewicht von 340 g/l und einem Epoxyäquivalent von 143.

Beispiel 2
(Copolymerisat aus N-Methylolacrylamid, N-Vinylpyrrolidon und N,N'-Methylenbisacrylamid)

In einem zylindrischen Gefäss mit Kreuzbalkenrührer, Rückflusskühler, Thermometer und Stickstoffeinleitrohr wurden 900 ml Paraffinöl (DAB 7), 0,2 g des weiter oben beschriebenen Copolymerisates aus Maleinsäureanhydrid und Vinylstearyläther, 44,1 g N-Methylolacrylamid, 48,4 g N-Vinylpyrrolidon, 7,5 g N,N'-Methylenbisacrylamid, 2 g Azo-diisobuttersäurenitril, 126 ml Dimethylformamid und 48 ml Polyäthylenglykol (Molgewicht 400) vorgelegt. Unter Einleiten von Stickstoff wurde die Badtemperatur langsam auf 65 °C hochgeheizt, dann – wie in Beispiel 1 beschrieben – polymerisiert und aufgearbeitet.

Die Ausgebeute betrug 89,1 g (= 89,1% der Theorie) vernetztes Produkt.

Ein im wesentlichen gleiches Produkt wird erhalten, wenn als Dispersionsmittel ein entsprechendes Copolymerisat aus Maleinsäureanhydrid und Vinyldodecyläther eingesetzt wird.

Die Überführung in das epoxydgruppenhaltige Produkt erfolgte in der in Beispiel 1, Abschnitt 5 angegebenen Weise. Das perlförmige Produkt hatte ein Schüttgewicht von 367 g/l und ein Epoxyäquivalent von 320.

Beispiel 3
(Copolymerisat aus N-Methylolacrylamid, N-Vinyl-N-methylacetamid, und N,N'-Methylenbisacrylamid)

In der in Beispiel 1 beschriebenen Apparatur wurde unter den in diesem Beispiel 1 angegebenen Bedingungen folgendes System der Polymerisation unterworfen:

900 ml Paraffinöl, 0,2 g des in Beispiel 2 verwendeten Copolymerisats aus Maleinsäureanhydrid und Vinylstearyläther, 46,7 g N-Methylolacrylamid, 45,8 g N-Vinyl-N-methylacetamid, 7,5 g N,N'-Methylenbisacrylamid, 2,0 g Azo-diisobuttersäurenitril, 126 ml Dimethylformamid und 48 ml Polyäthylenglykol (Molgewicht 400).

Die Aufarbeitung erfolgte gleichfalls wie in Beispiel 1 beschrieben.

Die Ausbeute betrug 94 g (= 94% der Theorie) vernetztes Copolymerisat.

Die Überführung in das epoxydgruppenhaltige Produkt erfolgte in der in Beispiel 1, Abschnitt 5 angegebenen Weise. Das perlförmige Produkt hatte ein Schüttgewicht von 287 g/l und ein Epoxydäquivalent von 193.

Beispiel 4
(Copolymerisat aus N-Methylolacrylamid, N-Vinylpyrrolidon und N,N'-Divinyläthylenharnstoff)

In der Apparatur gemäss Beispiel 2 wurde unter den Bedingungen von Beispiel 1 folgendes System der Polymerisation unterworfen

900 ml Paraffinöl, 0,2 g des in Beispiel 2 verwendeten Copolymerisats aus Maleinsäureanhydrid und Vinylstearyläther, 40,5 g N-Methylolacrylamid, 44,5 g N-Vinylpyrrolidon, 15 g N,N'-Divinyläthylenharnstoff, 2,0 g Azo-diisobuttersäurenitril, 126 ml Dimethylformamid und 48 ml Polyäthylenglykol (Molgewicht 400).

Die Aufarbeitung erfolgte wie in Beispiel 1 beschrieben.

Ausbeute: 96 g (= 96% der Theorie) vernetztes Copolymerisat.

Die Überführung in das epoxydgruppenhaltige Produkt erfolgte in der in Beispiel 1, Abschnitt 5 angegebenen Weise. Das perlförmige Produkt hatte ein Schüttgewicht von 493 g/l und ein Epoxydäquivalent von 284.

Beispiel 5
(Copolymerisat aus N-Methylolacrylamid, N-Vinylpyrrolidon und Glyoxalbisacrylamid)

In der Apparatur gemäss Beispiel 2 wurde unter den Bedingungen von Beispiel 1 folgendes System der Polymerisation unterworfen:

900 ml Paraffinöl, 0,2 g Copolymerisat aus Maleinsäureanhydrid und Vinylstearyläther (wie in Beispiel 2), 44,1 g N-Methylolacrylamid, 48,4 g N-Vinylpyrrolidon, 7,5 g Glyoxalbisacrylamid, 2,0 g Azo-diisobuttersäurenitril, 126 g Dimethylformamid und 48 ml Polyäthylenglykol (Molgewicht 400).

Die Aufarbeitung erfolgte wie in Beispiel 1.

Ausbeute: 94 g (= 94% der Theorie) vernetztes Copolymerisat.

Die Überführung in das epoxydgruppenhaltige Produkt erfolgte in der in Beispiel 1, Abschnitt 5 angegebenen Weise. Das Produkt hatte ein Schüttgewicht von 340 g/l und ein Epoxydäquivalent von 442.

Beispiel 6
(Copolymerisat aus N-Methylolacrylamid, N-Vinylpyrrolidon und N,N',N''-Trisacryloylperhydrotriazin)

In der Apparatur gemäss Beispiel 2 wurde unter den Bedingungen von Beispiel 1 folgendes System der Polymerisation unterworfen:

900 ml Paraffinöl, 0,2 g Copolymerisat aus Maleinsäureanhydrid und Vinylstearyläther (wie in Beispiel 2), 45,3 g N-Methylolacrylamid, 49,7 g N-Vinylpyrrolidon, 5,0 g N,N',N''-Trisacryloylperhydrotriazin, 2 g Azo-diisobuttersäurenitril, 126 ml Dimethylformamid und 48 ml Polyäthylenglykol (Molgewicht 400).

Die Aufarbeitung erfolgte wie in Beispiel 1 beschrieben.

Ausbeute: 79 g (= 79% der Theorie) vernetztes Copolymerisat.

Die Überführung in das epoxydgruppenhaltige Produkt erfolgte in der in Beispiel 1, Abschnitt 5 angegebenen Weise. Das perlförmige Produkt hatte ein Schüttgewicht von 233 g/l und ein Epoxydäquivalent von 357.

Beispiel 7
(Copolymerisat aus N-Methylolacrylamid, N-Vinylpyrrolidon und N,N'-Methylenbisacrylamid)

In der Apparatur gemäss Beispiel 2 wurde unter den Bedingungen von Beispiel 1 folgendes System der Polymerisation unterworfen:

900 ml Paraffinöl, 0,2 g Copolymerisat aus Maleinsäureanhydrid und Vinylstearyläther (wie in Beispiel 2), 40,5 g N-Methylolacrylamid, 44,5 g N-Vinylpyrrolidon, 15 g N,N'-Methylenbisacrylamid, 2 g Azo-diisobuttersäurenitril, 126 g Dimethylformamid und 45 ml Äthylenglykol.

Die Aufarbeitung erfolgte wie in Beispiel 1.

Ausbeute: 96 g (= 96% der Theorie) an vernetztem Copolymerisat.

Die Überführung in das epoxydgruppenhaltige Produkt erfolgte in der in Beispiel 1, Abschnitt 5 angegebenen Weise. Das Produkt hatte ein Schüttgewicht von 553 g/l und ein Epoxydäquivalent von 211.

Beispiel 8
(Copolymerisat aus N-Methylolacrylamid, N-Vinylpyrrolidon und N,N'-Methylenbisacrylamid)

In der Apparatur gemäss Beispiel 2 wurde unter den Bedingungen von Beispiel 1 folgendes System der Polymerisation unterworfen:

900 ml Paraffinöl, 1 g Copolymerisat aus Maleinsäureanhydrid und Octadecen-(1) (wie in Beispiel 1), 40,5 g N-Methylolacrylamid, 44,5 g N-Vinylpyrrolidon, 15 g Methylenbisacrylamid, 2 g Azo-diisobuttersäurenitril und 45 ml Glycerintriacetat.

Die Polymerisationszeit betrug eine Stunde bei 70 °C und 4 Stunden bei 80 °C.

Die Aufarbeitung erfolgte wie in Beispiel 1 beschrieben.

Ausbeute: 98 g (= 98% der Theorie) vernetztes Copolymerisat.

Die Überführung in das epoxydgruppenhaltige Produkt erfolgte in der in Beispiel 1, Abschnitt 5 angegebenen Weise. Das perlförmige Produkt hatte ein Schüttgewicht von 240 g/l und ein Epoxydäquivalent von 214.

Beispiel 9
(Copolymerisat aus N-Methylolacrylamid, N-Vinylpyrrolidon und N,N'-Methylenbisacrylamid)

In der Apparatur gemäss Beispiel 2 wurde unter den Bedingungen von Beispiel 1 folgendes System der Polymerisation unterworfen:

900 ml Paraffinöl, 0,2 g Copolymerisat aus Maleinsäureanhydrid und Vinylstearyläther (gemäss Beispiel 2), 44,1 g N-Methylolacrylamid, 48,4 g N-Vinylpyrrolidon, 7,5 g N,N'-Methylenbisacrylamid, 2,0 g Ammoniumperoxydisulfat, 30 ml destilliertes Wasser und 106 ml Diacetonalkohol.

Die Polymerisationszeit betrug 5 Stunden bei 80 °C. Die Aufarbeitung erfolgte wie in Beispiel 1.

Ausbeute: 72 g (= 72% der Theorie).

Die Überführung in das epoxydgruppenhaltige Produkt erfolgte in der in Beispiel 1, Abschnitt 5 angegebenen Weise. Das perlförmige Produkt hatte ein Schüttgewicht von 180 mg/l und ein Epoxydäquivalent von 460.

Beispiel 10
(Copolymerisat aus N-(2-Hydroxyäthyl)-acrylamid, N-Vinylpyrrolidon und N,N'-Methylenbisacrylamid)

In einem Rundkolben mit Rührer, Thermometer, Stickstoffeinleitrohr und Rückflusskühler wurden 800 ml dünnflüssiges Paraffinöl DAB 7 (Dispersionsmittel), 2,0 g eines Copolymerisates aus Maleinsäureanhydrid und Octadecen-(1), (Molverhältnis 1:1, RSV-Wert 0,064 dl/g, gemessen in 0,6%iger Lösung in Toluol bei 25 °C), 43,3 g N-(2-Hydroxyäthyl)-acrylamid, 41,7 g N-Vinylpyrrolidon, 15 g N,N'-Methylenbisacrylamid, 2 g Azodiisobuttersäurenitril, 212 ml Dimethylformamid und 90 ml Polyäthylenglykol (Molekulargewicht etwa 400) vorgelegt.

Dieses Gemisch wurde dann unter Rühren langsam hochgeheizt. Bei ca. 65 °C begann die exotherme Polymerisationsreaktion, wobei die Temperatur auf ca. 80 °C anstieg. Diese Temperatur wurde mittels eines thermostatisierten Ölbades 1 Stunde gehalten, dann wurde zur Auspolymerisation die Badtemperatur vier Stunden auf 90 °C erhöht. Anschliessend wurde das Heizbad entfernt und der Ansatz unter Rühren auf 40 °C abkühlen gelassen. Danach wurde die Rührung abgestellt, worauf sich nach einiger Zeit das perlförmige Polymerisat absetzte. Die Hauptmenge des Paraffinöls wurde dann abgehebert und anschliessend der Rest über eine Nutsche abgesaugt. Das erhaltene Polymerisat wurde anschliessend mit Petrolether behandelt, um das anhaftende Paraffinöl zu entfernen. Anschliessend wurde mit Methanol, dann mit Aceton ausgerührt und schliesslich mit Aceton bei Siedetemperatur extrahiert, um die nicht umgesetzten Monomeren sowie den Dispergator herauszulösen. Schliesslich wurde das Polymerisat im Vakuumschrank über Nacht bei 50 °C getrocknet und gesiebt.

Die Ausbeute betrug >95 g (= >95% der Theorie) vernetztes Copolymerisat.

Im wesentlichen das gleiche Produkt wird erhalten, wenn ein entsprechendes Dispersionsstabilisator aus Maleinsäureanhydrid und Dodecen eingesetzt wird.

Es wurden 15 g der Siebfraktion 50 bis 100 µm 16 Stunden in 100 ml Epichlorhydrin gequollen, dann vier Stunden auf 115 °C erhitzt und nach dem Abkühlen auf 25 °C abgesaugt. Dann wurde zweimal mit jeweils 200 ml Aceton während 30 Minuten verrührt, abgesaugt und über Nacht im Vakuumtrockenschrank bei 40 °C unter Stickstoffüberlagerung aufbewahrt.

Die Auswaage ergab 14,3 g perlförmiges Produkt mit einem Schüttgewicht von 440 g/l und einem Epoxyäquivalent von 182.

Beispiel 11
(Copolymerisat aus N-(2-Hydroxyäthyl)-acrylamid, N-Vinylpyrrolidon und N,N'-Äthylenbisacrylamid)

In einem zylindrischen Gefäss, mit Kreuzbalkenrührer, Rückflusskühler, Thermometer und Stickstoffeinleitrohr wurden 900 ml Paraffinöl (DAB 7), 0,2 g des in Beispiel 1 beschriebenen Copolymerisates aus Maleinsäureanhydrid und Vinylstearyläther, 47,1 g N-(2-Hydroxyäthyl)-acrylamid, 45,4 g N-Vinylpyrrolidon, 7,5 g N,N'-Äthylenbisacrylamid, 2 g Azo-diisobuttersäurenitril, 126 ml Dimethylformamid und 48 ml Polyäthylenglykol (Molgewicht 400) vorgelegt. Unter Einleiten von Stickstoff wurde die Badtemperatur langsam auf 65 °C hochgeheizt, dann wie im Beispiel 1 beschrieben polymerisiert und aufgearbeitet.

Die Ausbeute betrug 92,3 g (= 92,3% der Theorie) vernetztes Produkt.

Die Überführung in das epoxydgruppenhaltige Produkt erfolgte in der in Beispiel 1, Abschnitt 5 angegebenen Weise. Das perlförmige Produkt hatte ein Schüttgewicht von 385 g/l und ein Epoxyäquivalent von 340.

Beispiel 12
(Copolymerisat aus N-(2-Hydroxyäthyl)-acrylamid, N-Vinylpyrrolidon und N,N'-Hexamethylenbisacrylamid)

In einem zylindrischen Gefäss, mit Kreuzbalkenrührer, Rückflusskühler, Thermometer und Stickstoffeinleitrohr wurden 900 ml Paraffinöl (DAB 7), 0,2 g des in Beispiel 1 beschriebenen Copolymerisates aus Maleinsäureanhydrid und Vinylstearyläther, 43,3 g N-(2-Hydroxyäthyl)-acrylamid, 41,7 g N-Vinylpyrrolidon, 15 g Hexamethylenbisacrylamid, 2 g Azo-diisobuttersäurenitril, 126 ml Dimethylformamid und 48 ml Polyäthylenglykol (Molgewicht 400) vorgelegt. Unter Einleiten von Stickstoff wurde die Badtemperatur langsam auf 65 °C hochgeheizt, dann – wie im Beispiel 1 beschrieben – polymerisiert und aufgearbeitet.

Die Ausbeute betrug 85,3 g (= 85,3% der Theorie) vernetztes Produkt.

Die Überführung in das epoxydgruppenhaltige Produkt erfolgte in der in Beispiel 1, Abschnitt 5 angegebenen Weise. Das perlförmige Produkt hatte ein Schüttgewicht von 420 g/l und ein Epoxyäquivalent von 280.

Beispiel 13
(Copolymerisat aus N-(2-Hydroxyäthyl)-acrylamid, N-Vinylpyrrolidon und N,N'-Divinyläthylenharnstoff)

In einem zylindrischen Gefäss mit Kreuzbalkenrührer, Rückflusskühler, Thermometer und Stickstoffeinleitrohr wurden 900 ml Paraffinöl (DAB 7), 0,2 g des in Beispiel 1 beschriebenen Copolymerisates aus Maleinsäureanhydrid und Vinylstearyläther, 43,3 g N-(2-Hydroxyäthyl)-acrylamid, 41,7 g N-Vinylpyrrolidon, 15,0 g N,N'-Divinyläthylenharnstoff, 2 g Azo-diisobuttersäurenitril, 126 ml Dimethylformamid und 48 ml Polyäthylenglykol (Molgewicht 400) vorgelegt. Unter Einleiten von Stickstoff wurde die Badtemperatur langsam auf 65 °C hochgeheizt, dann – wie im Beispiel 1 beschrieben – polymerisiert und aufgearbeitet.

Die Ausbeute betrug 87,3 g (= 87,3% der Theorie) vernetztes Produkt.

Die Überführung in das epoxydgruppenhaltige Produkt erfolgte in der in Beispiel 1, Abschnitt 5 angegebenen Weise. Das perlförmige Produkt hatte ein Schüttgewicht von 395 g/l und ein Epoxyäquivalent von 287.

II. Umsetzung der erfindungsgemässen Polymerträger mit biologisch aktiven Substanzen

Beispiel 14

Zu 0,2 g eines nach Beispiel 1 hergestellten Trägers wurden 1100 µl einer Penicillin-Acylase-Lösung (25 mg/ml, 243 U/ml), die 1-molar an Kaliumphosphat (Puffer) war und einen pH-Wert von 8,0 aufwies, gegeben. Nach der Fixierung während 72 Stunden wurden die Perlen mit 1-molarer Kochsalzlösung und mit Pufferlösung gründlich gewaschen. Die Ausbeute an nutschenfeuchtem Material betrug 678 mg mit 270 Units/g, gemessen am Autotitrator bei 37 °C und einem pH-Wert von 7,8 mit penicillinsaurem Kalium als Substrat.

Bezogen auf Trockengewicht waren das 916 Units/g. Nach Bilanzierung von Ausgangs- und Waschwasseraktivität blieb eine Fixierungsausbeute (= Aktivität auf Träger: angebotene Aktivität) von 69%. Der $\eta$-Wert betrug 0,82 ($\eta$ = gefundene Aktivität/angebotene Aktivität minus Waschwasser-Aktivität).

Beispiel 15

Zu 0,2 g eines nach Beispiel 2 hergestellten Trägers wurden 1000 µl einer Trypsin-Lösung (6,25 mg/ml, 345 U/ml), die $1{,}6 \times 10^{-2}$ molar an Benzamidin und 1 molar an Kaliumphosphat (Puffer) war und einen pH-Wert von 7,8 aufwies, gegeben. Nach der Fixierung während 72 Stunden wurden die Perlen mit 1 molarer Kochsalzlösung und mit Puffer-Lösung gründlich gewaschen. Die Ausbeute an nutschenfeuchtem Material betrug 589 mg mit 485 Units/g, gemessen am Autotitrator bei 37 °C und einem pH-Wert von 8,1 mit N'-Benzoyl-L-argininäthylesterhydrochlorid (BAEE) als Substrat. Bezogen auf Trockengewicht waren das 1429 Units/g. Nach Bilanzierung von Ausgangs- und Waschwasseraktivität blieb eine Fixierungsausbeute von 60%. Der η-Wert betrug 0,61.

Beispiel 16

Zu 0,2 g eines nach Beispiel 2 hergestellten Trägers wurden 1000 µl einer Urease-Lösung (30 mg/ml, 45,5 U/ml), die 1 molar an Kaliumphosphat (Puffer) war und einen pH-Wert von 8,0 aufwies, gegeben. Nach der Fixierung während 72 Stunden wurden die Perlen mit 1 molarer Kochsalzlösung und mit Pufferlösung gründlich gewaschen. Die Ausbeute an nutschenfeuchtem Material betrug 561 mg mit 47 Units/g, gemessen am Autotitrator bei 30 °C und einem pH-Wert von 6,1 mit Harnstoff als Substrat. Bezogen auf Trockengewicht waren das 132 Units/g. Nach Bilanzierung von Ausgangs- und Waschwasseraktivität blieb eine Fixierungsausbeute von 83%. Der η-Wert betrug 0,87.

Beispiel 17

Zu 0,2 g eines nach Beispiel 2 hergestellten Trägers wurden 1000 µl einer Penicillin-Acylase-Lösung (25 mg/ml, 243 U/ml), die 1 molar an Kaliumphosphat (Puffer) war und einen pH-Wert von 8,0 aufwies, gegeben. Nach der Fixierung während 72 Stunden wurden die Perlen mit 1 molarer Kochsalzlösung und mit Pufferlösung gründlich gewaschen. Die Ausbeute an nutschenfeuchtem Material betrug 597 mg mit 356 Units/g, gemessen am Autotitrator bei 37 °C und einem pH-Wert von 7,8 mit penicillinsaurem Kalium als Substrat. Bezogen auf Trockengewicht waren das 1063 Units/g. Die Fixierungsausbeute betrug 88%. Der η-Wert lag bei 0,90.

Beispiel 18

Zu 0,2 g eines nach Beispiel 11 hergestellten Trägers wurden 900 µl einer Penicillin-Acylase-Lösung (25 mg/ml, 280 U/ml), die 1-molar an Kaliumphosphat (Puffer) war und einen pH-Wert von 8,0 aufwies, gegeben. Nach der Fixierung während 72 Stunden wurden die Perlen mit 1-molarer Kochsalzlösung und mit Pufferlösung gründlich gewaschen. Die Ausbeute an nutschenfeuchtem Material betrug 665 mg mit 250 Units/g, gemessen am Autotitrator bei 37 °C und einem pH-Wert von 7,8 mit penicillinsaurem Kalium als Substrat.

Bezogen auf Trockengewicht waren das 831 Units/g. Nach Bilanzierung von Ausgangs- und Waschwasseraktivität blieb eine Fixierungsausbeute (= Aktivität auf Träger: angebotene Aktivitä) von 67%. Der η-Wert betrug 0,68.

Beispiel 19

Zu 0,2 g eines nach Beispiel 12 hergestellten Trägers wurden 700 µl einer Trypsin-Lösung (6,25 mg/ml, 440 U/ml), die $1{,}6 \times 10^{-2}$ molar an Benzamidin und 1 molar an Kaliumphosphat (Puffer) war und einen pH-Wert von 7,8 aufwies, gegeben. Nach der Fixierung während 72 Stunden wurden die Perlen mit 1 molarer Kochsalzlösung und mit Puffer-Lösung gründlich gewaschen. Die Ausbeute an nutschenfeuchtem Material betrug 657 mg mit 460 Units/g, gemessen am Autotitrator bei 37 °C und einem pH-Wert von 8,1 mit N'-Benzoyl-L-argininäthylesterhydrochlorid (BAEE) als Substrat. Bezogen auf Trockengewicht waren das 1200 Units/g. Nach Bilanzierung von Ausgangs- und Waschwasseraktivität blieb eine Fixierungsausbeute von 80%. Der η-Wert betrug 0,9.

Beispiel 20

Zu 0,2 g eines nach Beispiel 13 hergestellten Trägers wurden 1000 µl einer Penicillin-Acylase-Lösung (25 mg/ml, 293 U/ml), die 1 molar an Kaliumphosphat (Puffer) war und einen pH-Wert von 8,0 aufwies, gegeben. Nach der Fixierung während 72 Stunden wurden die Perlen mit 1 molarer Kochsalzlösung und mit Pufferlösung gründlich gewaschen. Die Ausbeute an nutschenfeuchtem Material betrug 661 mg mit 310 Units/g, gemessen am Autotitrator bei 37 °C und einem pH-Wert von 7,8 mit penicillinsaurem Kalium als Substrat. Bezogen auf Trockengewicht waren das 1025 Units/g. Die Fixierungsausbeute betrug 70%. Der η-Wert lag bei 0,95.

**Patentansprüche für die Vertragsstaaten BE CH DE FR GB IT LI LU NL SE**

1. Polymerisat, bestehend im wesentlichen aus

a) Einheiten, die sich von mindestens einem Monomeren der Formel

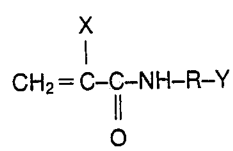

(I)

ableiten, in der X Wasserstoff oder Methyl ist, R einen aliphatischen Kohlenwasserstoffrest mit 1 bis 12 Kohlenstoffatomen bedeutet und Y für OH oder $NH_2$ steht, und aus Einheiten, die sich von weiteren Monomeren ableiten, die mit Monomeren der Formel (I) copolymerisierbar sind, wobei die mittlere Teilchengrösse der Polymerisat-Teilchen im Bereich von 20 bis 800 µm liegt, dadurch gekennzeichnet, dass als Einheiten von weiteren Monomeren solche vorhanden sind, die sich

b) von mindestens einem Monomeren mit hydrophilen Gruppen und

c) von mindestens einem Monomeren mit vernetzenden Gruppen ableiten und

dass die Polymerisat-Teilchen im wesentlichen kugelförmige Gestalt und einen mittleren Porendurchmesser von 5 bis 2000 nm aufweisen.

2. Polymerisat nach Anspruch 1, dadurch gekennzeichnet, dass der mittlere Porendurchmesser 10 bis 1000 nm beträgt.

3. Polymerisat nach Anspruch 1 oder Anspruch 2, dadurch gekennzeichnet, dass R in der Formel (I) ein aliphatischer Kohlenwasserstoffrest mit 1 bis 6 Kohlenstoffatomen und Y eine endständige (primäre) OH-Gruppe ist.

4. Polymerisat nach Anspruch 3, dadurch gekennzeichnet, dass R Methylen ist.

5. Polymerisat nach einem oder mehreren der Ansprüche 1 bis 4, dadurch gekennzeichnet, dass es aufgrund des Einbaus von vernetzten Monomereinheiten vernetzt ist.

6. Polymerisat nach einem oder mehreren der Ansprüche 1 bis 5, dadurch gekennzeichnet, dass es Einheiten enthält, die sich von Monomeren mit hydrophilen Gruppen ableiten.

7. Polymerisat nach einem oder mehreren der Ansprüche 1 bis 6, dadurch gekennzeichnet, dass zumindest ein Teil der Reste Y mit einem Spacer umgesetzt ist.

8. Polymerisat nach Anspruch 7, dadurch gekennzeichnet, dass der Spacer Epoxyd-Einheiten in einer Menge von 0,1 bis 20 Mol-%, bezogen auf das Gesamtpolymere, einführt.

9. Polymerisat nach einem oder mehreren der Ansprüche 1 bis 8, bestehend im wesentlichen aus 10 bis 80 Mol-% von Einheiten, die sich von Monomeren der Formel (I) ableiten, aus 20 bis 50 Mol-% von Einheiten, die sich von Monomeren mit hydrophilen Gruppen ableiten und aus 1 bis 50 Mol-% von Einheiten, die sich von Monomeren mit vernetzenden Gruppen ableiten.

10. Verfahren zur Herstellung der Polymerisate nach einem oder mehreren der Ansprüche 1 bis 9 durch Polymerisieren von

a) Verbindungen der Formel

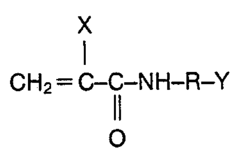

(I)

in der X, R und Y die obige Bedeutung haben, mit damit copolymerisierbaren weiteren Monomeren, wobei die Polymerisation in einem flüssigen Dispersionsmittel, das unter den Polymerisationsbedingungen die Monomeren und das Polymerisat nicht löst, in Gegenwart eines radikalisch wirksamen Initiators und eines Dispersionsstabilisators durchgeführt wird, dadurch gekennzeichnet, dass als weitere Monomere b) mindestens ein Monomer mit hydrophilen Gruppen und c) mindestens ein Monomer mit vernetzenden Gruppen eingesetzt werden und dass als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen α-Olefin mit 8 bis 30 C-Atomen verwendet wird.

11. Verfahren nach Anspruch 10, dadurch gekennzeichnet, dass der Dispersionsstabilisator in Mengen von 0,005 bis 10 Gew.-%, bezogen auf das Monomerengemisch, verwendet wird.

12. Verfahren nach Anspruch 10 oder 11, dadurch gekennzeichnet, dass der Dispersionsstabilisator ein alternierendes Copolymerisat ist.

13. Verfahren nach einem oder mehreren der Ansprüche 10 bis 12, dadurch gekennzeichnet, dass der RSV-Wert des als Dispersionsstabilisator eingesetzten Copolymerisates zwischen 0,01 und 1,0 dl/g (gemessen in 0,6%iger Lösung in Toluol bei 25 °C) liegt.

14. Verfahren nach einem oder mehreren der Ansprüche 10 bis 13, dadurch gekennzeichnet, dass der Vinylalkyläther Vinylstearyläther und das längerkettige α-Olefin Octadecen (1) ist.

15. Verfahren nach einem oder mehreren der Ansprüche 10 bis 14, dadurch gekennzeichnet, dass als flüssige Dispersionsmittel Kohlenwasserstoffe mit 6 bis 20 C-Atomen oder Paraffinöl dünnflüssig eingesetzt werden.

16. Verfahren nach einem oder mehreren der Ansprüche 10 bis 15, dadurch gekennzeichnet, dass das Polymerisationssystem zur Erhöhung der Porosität des Perlpolymerisates Stoffe enthält, die sich in den Monomeren lösen oder mit ihnen mischbar sind und im Dispersionsmittel unlöslich sind.

17. Verwendung der Polymerisate nach einem oder mehreren der Ansprüche 1 bis 9, vorzugsweise nach Umsatz mit Spacern, zur Herstellung trägergebundener, biologisch aktiver Substanzen.

18. Verwendung nach Anspruch 17, dadurch gekennzeichnet, dass die biologisch aktiven Substanzen Enzyme sind.

19. Ausführungsform nach einem oder mehreren der Ansprüche 1–16, dadurch gekennzeichnet, dass als Monomere b) N-Vinylpyrrolidon, (Meth)-Acrylamid, (Meth)Acrylsäurealkylester mit jeweils 2 bis 6 C-Atomen in der Alkylgruppe, Hydroxyalkylester der (Meth)Acrylsäure mit 2 bis 6 C-Atomen in der Alkylgruppe, N-Vinyl-N-alkylacetamid, ($C_1$–$C_4$-Alkyl), Vinylacetat oder Vinylencarbonat eingesetzt werden.

**Patentansprüche für den Vertragsstaat AT**

1. Verfahren zur Herstellung von Polymerisaten, bestehend im wesentlichen aus Einheiten, die sich

a) von Monomeren der Formel

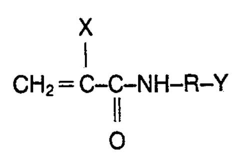

(I)

ableiten, in der X Wasserstoff oder Methyl ist, R einen aliphatischen Kohlenwasserstoffrest mit 1 bis 12 Kohlenstoffatomen bedeutet und Y für OH oder $NH_2$ steht, und aus Einheiten, die sich von weiteren Monomeren ableiten, die mit Monomeren der Formel (I) copolymerisierbar sind, wobei die mittlere Teilchengrösse der Polymerisat-Teilchen im Bereich von 20 bis 800 µm liegt und die Polymerisat-Teilchen im wesentlichen kugelförmige Gestalt und einen mittleren Porendurchmesser von 5 bis 2000 nm aufweisen, durch Polymerisation von Monomeren gemäss der Formel (I) mit damit copolymerisierbaren weiteren Monomeren, wobei die Polymerisation in einem flüssigen Dispersionsmittel, das unter den Polymerisationsbedingungen die Monomeren und das Polymerisat nicht löst, in Gegenwart eines radikalisch wirksamen Initiators und eines Dispersionsstabilisators durchgeführt wird, dadurch gekennzeichnet, dass als weitere Monomere b) mindestens ein Monomer mit hydrophilen Gruppen und c) mindestens ein Monomer mit vernetzenden Gruppen eingesetzt werden und dass als Dispersionsstabilisator ein Copolymerisat aus Maleinsäureanhydrid und einem Vinylalkyläther mit 6 bis 30 C-Atomen in der Alkylgruppe oder einem Vinylester mit 6 bis 30 C-Atomen in der Carbonsäuregruppe oder einem längerkettigen α-Olefin mit 8 bis 30 C-Atomen verwendet wird.

2. Verfahren nach Anspruch 1, dadurch gekennzeichnet, dass der Dispersionsstabilisator in Mengen von 0,005 bis 10 Gew.-%, bezogen auf das Monomerengemisch, verwendet wird.

3. Verfahren nach Anspruch 1 und/oder 2, dadurch gekennzeichnet, dass der Dispersionsstabilisator ein alternierendes Copolymerisat ist.

4. Verfahren nach einem oder mehreren der Ansprüche 1 bis 3, dadurch gekennzeichnet, dass der RSV-Wert des als Dispersionsstabilisator eingesetzten Copolymerisates zwischen 0,01 und 1,0 dl/g (gemessen in 0,6%iger Lösung in Toluol bei 25 °C) liegt.

5. Verfahren nach einem oder mehreren der Ansprüche 1 bis 4, dadurch gekennzeichnet, dass der Vinylalkyläther Vinylstearyläther und das längerkettige α-Olefin Octadecen (1) ist.

6. Verfahren nach einem oder mehreren der Ansprüche 1 bis 5, dadurch gekennzeichnet, dass als flüssige Dispersionsmittel Kohlenwasserstoffe mit 6 bis 20 C-Atomen oder Paraffinöl dünnflüssig eingesetzt werden.

7. Verfahren nach einem oder mehreren der Ansprüche 1 bis 6, dadurch gekennzeichnet, dass das Polymerisationssystem zur Erhöhung der Porosität des Perlpolymerisates Stoffe enthält, die sich in den Monomeren lösen oder mit ihnen mischbar sind und im Dispersionsmittel unlöslich sind.

8. Verfahren nach einem oder mehreren der Ansprüche 1 bis 7, dadurch gekennzeichnet, dass als weiteres Monomeres ein vernetzend wirkendes Monomeres eingesetzt wird.

9. Verfahren nach einem oder mehreren der Ansprüche 1 bis 8, dadurch gekennzeichnet, dass als weiteres Monomeres ein solches Monomeres eingesetzt wird, das hydrophile Gruppen aufweist.

10. Verwendung der Polymerisate, erhalten nach einem oder mehreren der Ansprüche 1 bis 9, vorzugsweise nach Umsatz mit Spacern, zur Herstellung trägergebundener, biologisch aktiver Substanzen.

11. Verwendung nach Anspruch 10, dadurch gekennzeichnet, dass die biologisch aktiven Substanzen Enzyme sind.

12. Verfahren nach einem oder mehreren der Ansprüche 1 bis 9, dadurch gekennzeichnet, dass als Monomere b) N-Vinylpyrrolidon, (Meth)-Acrylamid, (Meth)Acrylsäurealkylester mit jeweils 2 bis 6 C-Atomen in der Alkylgruppe, Hydroxyalkylester der (Meth)Acrylsäure mit 2 bis 6 C-Atomen in der Alkylgruppe, N-Vinyl-N-alkylacetamid, ($C_1$–$C_4$-Alkyl), Vinylacetat oder Vinylencarbonat eingesetzt werden.

**Claims for the contracting states: BE/CH/DE/FR/GB/IT/LI/LU/NL/SE**

1. A polymer substantially composed of

a) units derived from at least one monomer of the formula

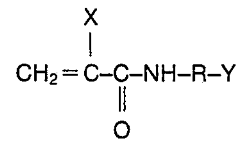

(I)

in which X is hydrogen or methyl, R denotes an aliphatic hydrocarbon radical having 1 to 12 carbon atoms, and Y represents OH or $NH_2$, and of units which are derived from further monomers which can be copolymerized with monomers of the formula (I), the mean particle size of the polymer particles being in the range from 20 to 800 µm, wherein such units are present as units of further monomers derived

b) from at least one monomer containing hydrophilic groups and

c) from at least one monomer containing crosslinking groups and wherein the polymer particles have an substantially spherical shape and a mean pore diameter of 5 to 2,000 nm.

2. The polymer as claimed in claim 1, which has a mean pore diameter of 10 to 1,000 nm.

3. The polymer as claimed in claim 1 or claim 2, wherein R in the formula (I) is an aliphatic hydrocarbon radical having 1 to 6 carbon atoms, and Y is a terminal (primary) OH group.

4. The polymer as claimed in claim 3, wherein R is methylene.

5. The polymer as claimed in one or more of claims 1 to 4, which is crosslinked by reason of the incorporation of crosslinked monomer units.

6. The polymer as claimed in one or more of claims 1 to 5, which contains units derived from monomers having hydrophilic groups.

7. The polymer as claimed in one or more of claims 1 to 6, wherein at least some of the radicals Y have been reacted with a spacer.

8. The polymer as claimed in claim 7, wherein the spacer introduces epoxide units in an amount of 0.1 to 20 mole-% relative to the total polymer.

9. The polymer as claimed in one or more of claims 1 to 8, which is essentially composed of 10 to 80 mole-% of units which derive from monomers of the formula (I), of 20 to 50 mole-% of units

which derive from monomers having hydrophilic groups, and of 1 to 50 mole-% of units which derive from monomers having crosslinking groups.

10. A process for the preparation of the polymer as claimed in one or more of claims 1 to 9 by polymerization of

a) compounds of the formula

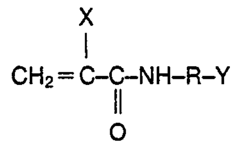
(I)

in which X, R and Y have the abovementioned meaning, with further monomers which can be copolymerized with it, the polymerization being carried out in a liquid dispersing agent which, under the polymerization conditions, does not dissolve the monomers and the polymer, in the presence of a radical initiator and a dispersion stabilizer, which comprises employing, as further monomers,

b) at least one monomer containing hydrophilic groups and

c) at least one monomer containing crosslinking groups and using, as the dispersion stabilizer, a copolymer of maleic anhydride and a vinyl alkyl ether having 6 to 30 carbon atoms in the alkyl group, or a vinyl ester having 6 to 30 carbon atoms in the carboxylic acid group, or a relatively long-chain α-olefin having 8 to 30 carbon atoms.

11. The process as claimed in claim 10, wherein the dispersion stabilizer is used in amounts of 0.005 to 10% by weight, based on the mixture of monomers.

12. The process as claimed in claim 10 or 11, wherein the dispersion stabilizer is an alternating copolymer.

13. The process as claimed in one or more of claims 10 to 12, wherein the RSV value of the copolymer employed as the dispersion stabilizer is between 0.01 and 1.0 dl/g (measured in 0.6% strength solution in toluene at 25 °C).

14. The process as claimed in one or more of claims 10 to 13, wherein the vinyl alkyl ether is vinyl stearyl ether, and the relatively long-chain α-olefin is 1-octadecene.

15. The process as claimed in one or more of claims 10 to 14, wherein hydrocarbons having 6 to 20 carbon atoms or low viscosity liquid paraffin are employed as the liquid dispersing agents.

16. The process as claimed in one or more of claims 10 to 15, wherein, to increase the porosity of the bead polymer, the polymerization system contains substances which are soluble in the monomers or are miscible with them and are insoluble in the dispersing agent.

17. The use of the polymer as claimed in one or more of claims 1 to 9, preferably after reaction with spacers, for the preparation of carrier-bound biologically active substances.

18. The use as claimed in claim 17, wherein the biologically active substances are enzymes.

19. An embodiment as claimed in one or more of claims 1 to 16, wherein the monomers b) employed are N-vinylpyrrolidone, (meth)acrylamide, and alkyl (meth)acrylates each having 2 to 6 carbon atoms in the alkyl group, hydroxyalkyl (meth)-acrylates having 2 to 6 carbon atoms in the alkyl group, N-vinyl-N-alkylacetamide ($C_1$–$C_4$-alkyl), vinyl acetate or vinylene carbonate.

**Claims for the contracting state AT**

1. A process for the preparation of a polymer substantially composed of units derived from

a) monomers of the formula

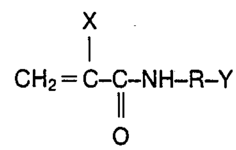
(I)

in which X is hydrogen or methyl, R denotes an aliphatic hydrocarbon radical having 1 to 12 carbon atoms, and Y represents OH or $NH_2$, and of units which are derived from further monomers which can be copolymerized with monomers of the formula (I), the mean particle size of the polymer particles being in the range from 20 to 800 µm and the polymer particles having an substantially spherical shape and a mean pore diameter of 5 to 2,000 nm, by polymerization of monomers of the formula (I) with further monomers which can be copolymerized with it, the polymerization being carried out in a liquid dispersing agent which, under the polymerization conditions, does not dissolve the monomers and the polymer, in the presence of a radical initiator and a dispersion stabilizer, which comprises employing, as further monomers,

b) at least one monomer containing hydrophilic groups and

c) at least one monomer containing crosslinking groups and using, as the dispersion stabilizer, a copolymer of maleic anhydride and a vinyl alkyl ether having 6 to 30 carbon atoms in the alkyl group, or a vinyl ester having 6 to 30 carbon atoms in the carboxylic acid group, or a relatively long-chain α-olefin having 8 to 30 carbon atoms.

2. The process as claimed in claim 1, wherein the dispersion stabilizer is used in amounts of 0.005 to 10% by weight, based on the mixture of monomers.

3. The process as claimed in claim 1 and/or 2, wherein the dispersion stabilizer is an alternating copolymer.

4. The process as claimed in one or more of claims 1 to 3, wherein the RSV value of the copolymer employed as the dispersion stabilizer is between 0.01 and 1.0 dl/g (measured in 0.6% strength solution in toluene at 25 °C).

5. The process as claimed in one or more of claims 1 to 4, wherein the vinyl alkyl ether is vinyl stearyl ether, and the relatively long-chain α-olefin is 1-octadecene.

6. The process as claimed in one or more of claims 1 to 5, wherein hydrocarbons having 6 to 20 carbon atoms or low viscosity liquid paraffin are employed as the liquid dispersing agents.

7. The process as claimed in one or more of claims 1 to 6, wherein, to increase the porosity of the bead polymer, the polymerization system contains substances which are soluble in the monomers or are miscible with them and are insoluble in the dispersing agent.

8. The process as claimed in one or more of claims 1 to 7, wherein the further monomer employed is a monomer which causes crosslinking.

9. The process as claimed in one or more of claims 1 to 8, wherein the further monomer employed is a monomer containing hydrophilic groups.

10. The use of the polymer obtained as claimed in one or more of claims 1 to 9, preferably after reaction with spacers, for the preparation of carrier-bound biologically active substances.

11. The use as claimed in claim 10, wherein the biologically active substances are enzymes.

12. The process as claimed in one or more of claims 1 to 9, wherein the monomers b) employed are N-vinylpyrrolidone, (meth)acrylamide, and alkyl (meth)acrylates each having 2 to 6 carbon atoms in the alkyl group, hydroxyalkyl (meth)acrylates having 2 to 6 carbon atoms in the alkyl group, N-vinyl-N-alkylacetamide ($C_1$–$C_4$-alkyl), vinyl acetate or vinylene carbonate.

**Revendications pour les Etats contractants: BE/CH/DE/FR/GB/IT/LI/LU/NL/SE**

1. Polymère substantiellement constitué:

a) de motifs dérivant d'au moins un monomère répondant à la formule I:

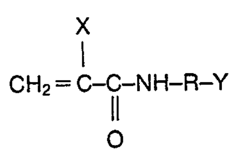

(I)

dans laquelle X représente l'hydrogène ou un méthyle, R représente un radical hydrocarboné aliphatique contenant de 1 à 12 atomes de carbone, et Y représente –OH ou –$NH_2$, ou de motifs dérivant d'autres monomères copolymérisables avec les monomères de formule I,

la granularité moyenne du polymère étant située dans l'intervalle allant de 20 à 800 µm, polymère caractérisé en ce qu'il contient comme autres motifs:

b) des motifs dérivant d'au moins un monomère à radicaux hydrophiles et

c) des motifs dérivant d'au moins un monomère à radicaux réticulants,

et en ce que les particules du polymère sont substantiellement de forme sphérique et ont un diamètre moyen de pores de 5 à 2 000 nm.

2. Polymère selon la revendication 1 caractérisé en ce que le diamètre moyen des pores est compris entre 10 et 1 000 nm.

3. Polymère selon l'une des revendications 1 et 2 caractérisé en ce que, dans la formule I, le symbole R représente un radical hydrocarboné aliphatique contenant de 1 à 6 atomes de carbone et le symbole Y représente un radical hydroxy (primaire) terminal.

4. Polymère selon la revendication 3 caractérisé en ce que R représente un radical méthylène.

5. Polymère selon l'une quelconque des revendications 1 à 4, caractérisé en ce qu'il est réticulé grâce à la présence, dans ce polymère, de motifs monomères réticulés.

6. Polymère selon l'une quelconque des revendications 1 à 5, caractérisé en ce qu'il contient des motifs qui dérivent de monomères porteurs de radicaux hydrophiles.

7. Polymère selon l'une quelconque des revendications 1 à 6, caractérisé en ce que les radicaux Y ont réagi, au moins en partie, avec un composé jouant le rôle d'«écarteur».

8. Polymère selon la revendication 7 caractérisé en ce que l'écarteur introduit des motifs d'époxyde en une quantité de 0,1 à 20% en moles par rapport au polymère total.

9. Polymère selon l'une quelconque des revendications 1 à 8, polymère qui est essentiellement constitué de 10 à 80% en moles de motifs dérivant de monomères de formule I, de 20 à 50% en moles de motifs dérivant de monomères porteurs de radicaux hydrophiles, et de 1 à 50% en moles de motifs dérivant de monomères porteurs de radicaux réticulants.

10. Procédé pour préparer des polymères selon l'une quelconque des revendications 1 à 9, par polymérisation a) de composés répondant à la formule I:

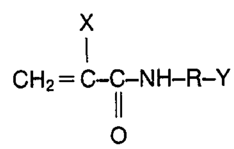

(I)

dans laquelle X, R et Y ont les significations précédemment données, avec au moins un autre monomère copolymérisable avec les précédents, cette polymérisation étant effectuée dans un milieu de dispersion liquide ne dissolvant, dans les conditions de la polymérisation, ni les monomères ni le polymère, en présence d'un amorceur radicalaire et d'un stabilisant de dispersion, procédé caractérisé en ce qu'on utilise, comme autres monomères, b) au moins un monomère à radicaux hydrophiles et c) au moins un monomère à radicaux réticulants, et en ce qu'on utilise, comme stabilisant de dispersion, un copolymère de l'anhydride maléique et d'un alcoxy-éthylène dont la partie alkyle contient de 6 à 30 atomes de carbone ou d'un ester vinylique dont le radical d'acide carboxylique contient de 6 à 30 atomes de carbone ou d'une α-oléfine à longue chaîne qui contient de 8 à 30 atomes de carbone.

11. Procédé selon la revendication 10 caractérisé en ce que le stabilisant de dispersion est

utilisé en une quantité de 0,005 à 10% en poids par rapport au mélange des monomères.

12. Procédé selon l'une des revendications 10 et 11, caractérisé en ce que le stabilisant de dispersion est un copolymère à distribution alternée.

13. Procédé selon l'une quelconque des revendications 10 à 12, caractérisé en ce que la viscosité spécifique réduite du copolymère utilisé comme stabilisant de dispersion est comprise entre 0,01 et 1,0 dl/g (mesurée à 25 °C sur une solution à 0,6% dans du toluène).

14. Procédé selon l'une quelconque des revendications 10 à 13, caractérisé en ce que l'oxyde de vinyle et d'alkyle est l'oxyde de vinyle et de stéaryle, et l'α-oléfine à longue chaîne est l'octadécène-1.

15. Procédé selon l'une quelconque des revendications 10 à 14, caractérisé en ce qu'on utilise, comme milieux de dispersion liquides, des hydrocarbures qui contiennent de 6 à 20 atomes de carbone ou une huile de paraffine très fluide.

16. Procédé selon l'une quelconque des revendications 10 à 15, caractérisé en ce que le mélange de polymérisation contient, pour que la porosité (dimension des pores) du polymère en perles soit plus grande, des matières qui se dissolvent dans les monomères ou qui sont miscibles avec les monomères mais qui sont insolubles dans le milieu de dispersion.

17. Application des polymères selon l'une quelconque des revendications 1 à 9, de préférence après réaction avec des composés «écarteurs», à la préparation de substances bio-actives immobilisées (fixées sur un support).

18. Application selon la revendication 17 caractérisée en ce que les substances bio-actives sont des enzymes.

19. Forme de réalisation suivant une ou plusieurs des revendications 1 à 16, caractérisée en ce que l'on utilise comme monomère (b) la N-vinylpyrrolidone, le (méth)-acrylamide, un (méth)-acrylate d'alkyle en $C_2$–$C_6$, un (méth)-acrylate d'hydroxyalkyle en $C_2$–$C_6$, un N-vinyl-N-alkylacétamide à alkyle en $C_1$–$C_4$, l'acétate de vinyle ou le carbonate de vinylène.

**Revendications pour l'Etat contractant: Autriche**

1. Procédé pour préparer des polymères substantiellement constitués de motifs qui dérivent:

a) de motifs d'au moins un monomère répondant à la formule I:

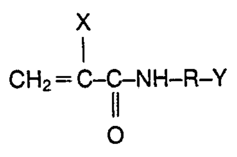

(I)

dans laquelle X représente l'hydrogène ou un radical méthyle, R représente un radical hydrocarboné aliphatique contenant de 1 à 12 atomes de carbone, et Y représente –OH, ou –$NH_2$, et de motifs qui dérivent d'autres monomères copolymérisables avec des monomères de formule I, la granularité moyenne du polymère étant située dans l'intervalle allant de 20 à 800 µm et les particules du polymère ayant une forme substantiellement sphérique et un diamètre moyen des pores de 5 à 2 000 nm, par polymérisation de monomères de formule I avec d'autres monomères copolymérisables avec des monomères de formule I, la polymérisation étant effectuée dans un milieu de dispersion liquide qui, dans les conditions de la polymérisation, ne dissout ni les monomères ni le polymère, en présence d'un amorceur radicalaire et d'un stabilisant de dispersion, procédé caractérisé en ce qu'on utilise, comme autres monomères, b) au moins un monomère porteur de radicaux hydrophiles et c) au moins un monomère porteur de radicaux réticulants, et en ce qu'on utilise, comme stabilisant de dispersion, un copolymère de l'anhydride maléique et d'un oxyde de vinyle et d'alkyle dont le radical alkyle contient de 6 à 30 atomes de carbone ou d'un ester vinylique d'un acide carboxylique contenant de 6 à 30 atomes de carbone ou d'une α-oléfine à longue chaîne qui contient de 8 à 30 atomes de carbone.

2. Procédé selon la revendication 1 caractérisé en ce que le stabilisant de dispersion est utilisé en une quantité de 0,005 à 10% en poids par rapport au mélange des monomères.

3. Procédé selon l'une des revendications 1 et 2, caractérisé en ce que le stabilisant de dispersion est un copolymère à distribution alternée.

4. Procédé selon l'une quelconque des revendications 1 à 3, caractérisé en ce que la viscosité spécifique réduite du copolymère utilisé comme stabilisant de dispersion est comprise entre 0,01 et 1,0 dl/g (mesurée à 25 °C sur une solution à 0,6% dans du toluène).

5. Procédé selon l'une quelconque des revendications 1 à 4, caractérisé en ce que l'oxyde de vinyle et d'alkyle est l'oxyde de vinyle et de stéaryle, et l'α-oléfine à longue chaîne est l'octadécène-1.

6. Procédé selon l'une quelconque des revendications 1 à 5, caractérisé en ce qu'on utilise comme milieux de dispersion liquides des hydrocarbures contenant de 6 à 20 atomes de carbone, ou une huile de paraffine très fluide.

7. Procédé selon l'une quelconque des revendications 1 à 6, caractérisé en ce que le mélange de polymérisation contient, pour que la porosité (dimension des pores) du polymère en perles soit plus grande, des matières qui se dissolvent dans les monomères ou qui sont miscibles avec les monomères mais qui sont insolubles dans le milieu de dispersion.

8. Procédé selon l'une quelconque des revendications 1 à 7, caractérisé en ce qu'on utilise, comme autre monomère, un monomère à action réticulante.

9. Procédé selon l'une quelconque des revendications 1 à 8, caractérisé en ce qu'on utilise, comme autre monomère, un monomère qui porte des radicaux hydrophiles.

10. Application des polymères qui ont été obtenus selon l'une quelconque des revendications 1 à 9, de préférence après réaction avec des composés «écarteurs», à la préparation de substances bio-actives immobilisées (fixées à un support).

11. Application selon la revendication 10, caractérisée en ce que les substances bio-actives sont des enzymes.

12. Procédé selon l'une quelconque des revendications 1 à 9, caractérisé en ce qu'on utilise, comme monomère b), la N-vinyl-pyrrolidone, l'acrylamide, le méthacrylamide, un acrylate ou méthacrylate d'alkyle dont le radical alkyle contient de 2 à 6 atomes de carbone, un acrylate ou méthacrylate d'hydroxy-alkyle dont le radical alkyle contient de 2 à 6 atomes de carbone, un N-vinyl-N-alkyl-acétamide dont le radical alkyle contient de 1 à 4 atomes de carbone, l'acétate de vinyle ou le carbonate de vinylène.